# DEMANDE DE BREVET EUROPEEN

(11) **EP 1 175 892 A1**
(43) Date de publication de la demande: **30.01.2002**
(21) Numéro de dépôt: 01401976.4
(22) Date de dépôt: 23.07.2001
(51) Int. Cl.: A61K 7/06, A61K 7/155

(54) **Utilisation d'antagonistes du récepteur des prostaglandines EP-3 comme agent cosmétique permettant d'atténuer ou d'arrêter la chute des cheveux et des poils**

(30) Priorité: 28.07.2000 FR 0009985
(71) Demandeur: L'OREAL, 75008 Paris (FR)
(72) Inventeur: Michelet, Jean-Francois, 94000 Creteil (FR); Mahe, Yann, 91390 Morsang-sur-Orge (FR); Bernard, Bruno, 92200 Neuilly-sur-Seine (FR)
(74) Mandataire: Casalonga, Axel

(57) **Abrégé**

L'invention concerne l'utilisation d'antagonistes du récepteur des prostaglandines EP-3 comme agent cosmétique permettant d'atténuer, de diminuer ou d'arrêter la chute des cheveux et des poils.

## Description

L'invention concerne l'utilisation d'antagonistes du récepteur des prostaglandines EP-3 comme agent cosmétique permettant d'atténuer, de diminuer ou d'arrêter la chute des cheveux et des poils.

L'homme a un capital de 100.000 à 150.000 cheveux et il est normal de perdre quotidiennement 50 à 100 cheveux. La maintenance de ce capital résulte essentiellement du fait que la vie d'un cheveu est soumise à un cycle pilaire au cours duquel le cheveu se forme, croît et tombe, avant d'être remplacé par un nouveau cheveu qui apparaît dans le même follicule.

On observe au cours d'un cycle pilaire successivement trois phases, à savoir: la phase anagène, la phase catagène et la phase télogène.

Au cours de la première phase, dite anagène, le cheveux passe par une période de croissance active associée à une intense activité mitotique au niveau du bulbe.

La deuxième phase, dite catagène, est transitoire, et elle est marquée par un arrêt de l'activité mitotique du bulbe. Au cours de cette phase, le cheveu subit une involution, le follicule s'atrophie et son implantation dermique apparaît de plus en plus haute.

La phase terminale, dite télogène, correspond à une période de repos du follicule et le cheveu finit par tomber. Après cette phase de repos un nouveau follicule est régénéré, sur place.

Ce processus de renouvellement physique permanent subit une évolution naturelle au cours du vieillissement, les cheveux deviennent plus fins, leurs cycles plus courts (M. Courtois et al., 1995, Br. J. Dermatol., 132: 86-93).

Dans presque tous les cas, la chute des cheveux survient chez des sujets prédisposés génétiquement; elle atteint plus particulièrement les hommes.

Cette chute des cheveux survient lorsque le processus de renouvellement physique est accéléré ou perturbé, c'est-à-dire que les phases de croissance sont raccourcies (Mr Courtois et al., 1994, Skin Pharmacol., 7: 84-89), le passage des cheveux en phase télogène est plus précoce et les cheveux tombent en plus grand nombre. Les cycles de croissance successifs aboutissent à des cheveux de plus en plus fins et de plus en plus courts, se transformant peu à peu en un duvet non pigmenté. Ce phénomène peut conduire à la calvitie.

On recherche depuis de nombreuses années dans l'industrie cosmétique ou pharmaceutique des compositions permettant de supprimer ou de réduire la chute des cheveux et notamment d'induire ou de stimuler la croissance des cheveux.

Dans cette optique, on a utilisé des composés tels que le 6-1-(pipéridinyl)-2,4-pyrimidine diamine-3-oxyde ou "Minoxidil". L'utilisation d'une lotion contenant un dérivé azolé et plus précisément le 1-acétyl-4-{4-[2-(2,4-dichlorophényl)-2-( 1H-imidazol-1-yl-méthyl)-1,3-dioxolan-4-ylméthoxy]-phényl} pipérazine pour le traitement de l'alopécie est décrite dans le brevet WO 92/00057.

Parallèlement, l'article "Growth regulation of primary human keratinocytes by prostaglandin E receptor EP₂ and EP₃ subtypes" de Konger et al. (Biochimica Biophysica Acta, 1401,1998, 221-224) décrit que les récepteurs des prostaglandines EP-3 jouent un rôle important dans la régulation de la croissance des kératinocytes de l'épiderme.

Néanmoins, il est bien connu que les programmes de différenciation des kératinocytes de l'épiderme et du follicule pileux sont clairement différents. Ainsi, il est connu que les marqueurs de différenciation telle que les kératines K1 et K10 ne sont pas exprimés dans le follicule pileux et en particulier dans la gaine externe (Lenoir et al., 1988, Dev. Biol. 130: 610-620); que la trichohyaline est exprimée dans le follicule pileux en particulier dans la gaine interne mais pas dans l'épiderme (O'Guin et al., 1992, J. Invest. Dermatol. 98: 24-32); et que la cyclooxygénase de type 1 n'est pas exprimée dans les kératinocytes du follicule pileux mais dans l'épiderme (Michelet. et al., 1997, J. Invest. Dermatol. 108: 205-209).

De plus, il est connu que les kératinocytes de l'épiderme et du follicule pileux se comportent de façon différente en réponse à un même agent pharmacologique. Ainsi, il est connu qu'in vivo le traitement de l'épiderme par l'acide rétinoïque induit une hyperplasie et une spongiose (Griffiths et al., 1993, J. Invest. Dermatol. 101: 325-328) tandis que le traitement du cuir chevelu induit une chute des cheveux (Berth-Jones et al., 1990, Br. J. Dermatol. 122:751-755), et qu'in vitro l'acide rétinoïque, selon la dose utilisée, favorise ou réduit la différenciation de l'épiderme (Asselineau et al., 1989, Dev. Biol. 133:32-335), tandis qu'il provoque l'arrêt de la croissance du follicule pileux (Billoni et al., 1997, Acta Dermatol.Venerol. 77: 350-355). Il est également connu que l'EGF induit une hyperplasie de l'épiderme et simultanément une régression du follicule pileux (Philip et al., 1985, J. Invest. Dermatol. 84: 172-175).

Par ailleurs, il est connu que le sulprostone est un agoniste du récepteur aux prostaglandines EP-3 dans le document "VII. International Union of Pharmacology Classification of Prostanoid Receptors: Properties, Distribution, and Structure of the Receptors and Their Subtypes" de Coleman et al., Pharmacological Reviews, 1994, Vol 46. Ce document précise que les récepteurs aux prostaglandines induisent une diminution du taux d'AMPc. Un exemple d'antagoniste du récepteur EP-3 est par exemple l'antagoniste non-prostanoïque L789106 décrit par MERCK FROOST (M. Labelle et al., 11^{th} International Conference on Advances in Prostaglandin and leukotriene research: Basic science and new clinical applications. Florence, du 4 au 8 Juin 2000)

Les prostaglandines sont des effecteurs biologiques issus d'acide gras polyinsaturés comme par exemple l'acide arachidonique pour PGA₂, PGE₂, PGF2α, TXA₂, ou encore l'acide dihomo γ linolénique pour PGE₁. Les prostaglandines sont impliquées dans de nombreux phénomènes de régulations physiologiques.

La demanderesse a découvert maintenant qu'en utilisant des antagonistes du récepteur des prostaglandines EP-3, on constatait de façon surprenante, une induction et une stimulation importantes de la croissance des cheveux et des poils et une action forte sur le ralentissement de la chute des cheveux et des poils.

La demanderesse a ainsi constaté que l'utilisation conforme à l'invention permettait d'obtenir un effet rapide, à une concentration faible avec/ou une fréquence d'application faible. Ces antagonistes sont capables d'arrêter la chute des cheveux et des poils et/ou d'augmenter la repousse des cheveux et des poils.

De plus, les antagonistes du récepteur des prostaglandines EP-3 de l'invention sont particulièrement peu toxiques et présentent une bonne conservation.

L'utilisation de ces antagonistes permet d'obtenir, notamment par rapport à celles antérieurement connues, des compositions plus efficaces pouvant être utilisées de façon particulièrement aisée, et permettant en outre une élimination facile des compositions par simple rinçage.

Les composés conformes à l'invention sont par ailleurs particulièrement appropriées sur le plan cosmétique et ne provoquent pas d'irritation du cuir chevelu, même après un contact prolongé, sans rinçage.

Ainsi l'invention a pour objet l'utilisation d'antagonistes du récepteur des prostaglandines EP-3 comme agent cosmétique ou dermatologique permettant d'atténuer, de diminuer ou d'arrêter la chute des cheveux et des poils. Ces antagonistes sont également capables d'augmenter la repousse des cheveux et des poils.

L'invention a également pour objet l'utilisation d'un antagoniste du récepteur des prostaglandines EP-3 dans une composition cosmétique ainsi que dans un procédé de traitement cosmétique destiné à atténuer, diminuer ou arrêter la chute des cheveux et des poils.

L'invention a pour objet principal une composition cosmétique ou dermatologique contenant au moins un antagoniste du récepteur des prostaglandines EP-3 dans un milieu cosmétiquement ou dermatologiquement acceptable.

Les récepteurs des prostaglandines EP-2 et/ou EP-4 sont des récepteurs des prostaglandines de la série E2. Ces récepteurs regroupent une famille de 4 représentants majeurs (EP1, EP2, EP3, EP4) et ont des activités tissulaires très variées.

Un antagoniste est un composé qui va se fixer sur un récepteur et qui ne va pas induire une réponse biologique, contrairement à l'agoniste qui va induire une réponse similaire à celle que l'on obtient avec le ligand naturel qui active cette réponse.

On entend par antagoniste des récepteurs des prostaglandines EP-3 des composés qui permettent d'atténuer, de diminuer ou de stopper la chute des cheveux et des poils. Ces antagonistes sont capables d'arrêter la chute des cheveux et des poils et/ou d'augmenter la repousse des cheveux et des poils.

On entend également par poils, les cils, les sourcils et tous les poils d'une manière générale.

Selon l'invention, ladite composition cosmétique peut contenir de 0,001 à 10% et de préférence de 0,1 à 5% d'antagonistes du récepteur des prostaglandines EP-3 en poids par rapport au poids de la composition.

Il est également possible d'utiliser en outre d'autres agents cosmétiques ou dermatologique destinés à arrêter la chute cheveux et des poils et/ou d'augmenter la repousse des cheveux et des poils dans les compositions cosmétiques définies précédemment comme par exemple les agonistes des récepteurs des prostaglandines EP-2 et/ou EP-4 dans des proportions allant de 0,001 à 10% et de préférence de 0,1 à 5% d'agonistes en poids par rapport au poids de la composition, ou encore des composés connus pour leur propriétés sur la chute et/ou la repousse des poils et/ou des cheveux comme par exemple le Minoxidil ou le 2,4-diamino pyrimidine 3-oxyde ou Aminexil.

Le milieu physiologiquement acceptable utilisé pour les compositions de l'invention est un milieu pouvant être constitué par de l'eau ou un mélange d'eau et d'un solvant ou un mélange de solvants. Les solvants sont choisis parmi les solvants organiques acceptables choisis plus particulièrement parmi les alcools mono- ou polyfonctionnels inférieurs en C₁-C₄ comme l'alcool éthylique, l'alcool isopropylique, l'alcool tertiobutylique, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

Les solvants, lorsqu'ils sont présents, le sont dans des proportions comprises entre 5 et 98 % en poids par rapport au poids total de la composition.

La composition peut également contenir en outre une phase grasse. Dans ce cas là, la phase grasse représente 0 à 50 % du poids total de la composition.

Ces compositions peuvent également renfermer:
- des oligosaccharides estérifiés tels que ceux décrits dans EP-A-0 064 012;
- des dérivés d'acide hexosaccharique tels que ceux décrits dans EP-A- 0 375 388, en particulier l'acide glucosaccharique;
- des inhibiteurs de glycosidase tels que ceux décrits dans EP-A-0 334 586, en particulier le D-glycaro 1,5-lactame;
- des inhibiteurs de glycosaminoglycanase et protéoglycanase tels que ceux cités dans EP-A-0 277 428, en particulier la L-galactono 1,4-lactone;
- des inhibiteurs de tyrosine kinase tels que ceux décrits dans EP-A-0 403 238, en particulier le 1-amido 1-cyano-(3,4-dihydroxyphényl)éthylène;
- des hyperémiants tels que:
   - les esters d'acide nicotinique dont plus particulièrement les nicotinates de benzyle et d'alkyle en C₁-C₆ et notamment le nicotinate de méthyle, de benzyle, ainsi que le nicotinate de tocophérol;
   - les bases de xanthine dont plus particulièrement la caféine et la théophilline;
   - la capsaicine;
- des filtres UV-A et UV-B comme les méthoxycinnamates, les dérivés de benzophénone;
- des inhibiteurs de la phosphodiestérase tels que la Visnadine®;
- des activateurs de l'adénine cyclase tels que le Forskolin;
- des antioxydants et capteurs de radicaux libres, en particulier
   - des radicaux OH tels que le DMSO;
   - 1'α-tocophérol, BHA, BHT;
   - la superoxyde dismutase (SOD);
- des agents antipelliculaires tels que l'omadine, l'octopirox;
- des agents hydratants tels que l'urée, la glycérine, l'acide lactique, les α-hydroxyacides, la thiamorpholinone et ses dérivés, des lactones;
- des agents antiséborrhériques tels que la S carboxyméthylcystéine, la S-benzylcystéamine et leurs dérivés, la thioxolone;
- des antiandrogènes et hormones tels que l'estriol, l'estradiol, la thyroxine, l'oxendolone, le diéthylstibelstrol;
- des rétinoïdes dont plus particulièrement l'acide t-trans rétinoïque appelé encore trétinoïne, l'isotrétinoïne, le rétinol ou la vitamine A et ses dérivés, tels que l'acétate, le palmitate ou le proprionate, le motretinide, l'étretinate, le t-trans rétinoate de zinc;
- des antibactériens choisis plus particulièrement parmi l'Irgasan, les macrolides, les pyranosides et les tetracyclines, et notamment l'erythromycine;
- des antagonistes de calcium dont on peut citer la Cinnarizine et le Diltiazem à titre d'exemple non limitatifs;
- des phospholipides tels que la lécithine;
- du diazoxyde (3-méthyl 7-chloro[2H]benzothiadiazine-1,2,4 dioxyde-1,1);
- des acides linoléïques et linoléniques;
- de l'anthraline et ses dérivés;
- de l'acide 5-alkanoyl salicylique et ses dérivés tels que décrits dans le brevet FR-25 81 542;
- des activateurs de pénétration tels que le THF, le 1,4-dioxane, l'acide oléique, la 2-pyrrolidone, le salicylate de benzyle, etc.,
- des vitamines ou provitamines tels que la β-carotène, la biotine, le panthénol et ses dérivés, la vitamine C, les vitamines B₂, B₄, B₆.

Ces compositions peuvent également contenir de l'AMP cyclique.

Ces compositions peuvent également contenir en outre des agents conservateurs, des agents stabilisants, des agents régulateurs de pH, des agents modificateurs de pression osmotique, des agents émulsifiants, des gélifiants et/ou épaississants classiques hydrophiles ou lipophiles; des actifs hydrophiles ou lipophiles; des conservateurs; des antioxydants; des parfums; des émulsionnants; des agents hydratants; des agents pigmentants; des dépigmentants; des agents kératolytiques; des vitamines; des émollients; des séquestrants; des tensio-actifs; des polymères; des agents alcanisants ou acidifiants; des charges; des agents anti-radicaux libres; des céramides; des filtres solaires; des répulsifs pour insectes; des agents amincissants; des matières colorantes; des bactéricides; des anti-pelliculaires.

Les compositions conformes à l'invention peuvent également contenir des agents tensio-actifs dont notamment ceux choisis parmi les agents tensio-actifs non ioniques et amphotères.

Parmi les tensio-actifs non ioniques, on citera les polyhydroxypropyléthers décrits notamment dans les brevets français n° 1477 048; 2 091 516; 2 169 787; 2 328 763; 2 574 786; les alkyl(C₈-C₉)phénols oxyéthylénés comportant de 1 à 100 moles d'oxyde d'éthylène et de préférence 5 à 35 moles d'oxyde d'éthylène; les alkylpolyglycosides de formule: CₙH₂ₙ₊₁ (C₆H₁₀O₅)ₓH dans laquelle n varie de 8 à 15 inclus et x de 1 à 10 inclus.

Parmi les agents tensio-actifs amphotères, on citera plus particulièrement les amphocarboxyglycinates et les amphocarboxypropionates définis dans le dictionnaire CTFA, 3ème édition, 1982, et vendus, notamment, sous la dénomination MIRANOL® par la Société MIRANOL.

On peut également utiliser des tensio-actifs cationiques et/ou anioniques.

Les composés conforme à l'invention peuvent également être introduits dans des supports gélifiés ou épaissis, tels que des supports essentiellement aqueux gélifiés par des hétérobiopolysaccharides, tels que la gomme de xanthane, les scléroglucanes ou les dérivés de cellulose, en particulier les éthers de cellulose, des supports hydroalcooliques gélifiés par des polyhydroxyéthylacrylates ou méthacrylates ou des supports essentiellement aqueux épaissis en particulier par des acides polyacryliques réticulés par un agent polyfonctionnel, tel que les Carbbopol vendus par la Société GOODRICH.

Les épaississants sont présents de préférence dans des proportions comprises entre 0,05 et 5% en poids et en particulier entre 0,2 et 3% en poids par rapport au poids total de la composition.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

La composition définie ci-dessus peut se présenter sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules.

La composition peut avoir un pH compris entre 3 et 8.

La composition peut avoir l'aspect d'une crème blanche ou colorée, d'une pommade, d'un lait, d'une lotion, d'un sérum, d'une pâte, d'une mousse ou d'un solide.

Ces compositions définis ci-dessus peuvent être appliquées sur les cheveux ou le cuir chevelu et peuvent s'appliquer, par exemple après un lavage du cuir chevelu et des cheveux à l'aide d'un shampooing.

L'invention a également pour objet l'utilisation d'antagonistes du récepteur des prostaglandines EP-3 comme agent cosmétique ou dermatologique permettant d'atténuer, de diminuer ou d'arrêter la chute cheveux et des poils. Ces antagonistes sont également capables d'augmenter la repousse des cheveux et des poils.

L'invention a aussi pour objet l'utilisation d'une composition telle que définie précédemment pour atténuer, de diminuer ou de stopper la chute des cheveux et des poils.

L'invention a encore pour objet un procédé de traitement cosmétique ou dermatologique destiné à atténuer, diminuer ou stopper la chute des cheveux et des poils, qui consiste à appliquer sur les cheveux ou les poils une quantité cosmétiquement ou dermatologiquement efficace d'antagonistes du récepteur des prostaglandines EP-3.

Un autre procédé de traitement cosmétique ou dermatologique destiné à atténuer, diminuer ou stopper la chute des cheveux et des poils, consiste à appliquer sur les cheveux ou les poils une composition cosmétique ou dermatologique telle que définie précédemment.

Les exemples qui suivent sont destinés à illustrer l'invention sans pour autant présenter un caractère limitatif.

### Exemple I:

| **LOTION ANTICHUTE** | |
|---|---|
| Antagoniste du récepteur | |
| des prostaglandines EP-3 | 0,5 g |
| Propylène glycol | 20 g |
| Ethanol 95° | 30 g |
| Eau qsp | 100 g |

Cette lotion est appliquée quotidiennement à raison de 10 ml sur le cuir chevelu et ce pendant 2 à 3 mois. On constate alors un net ralentissement de la chute quotidienne des cheveux ou des poils.

### Exemple II:

| **SHAMPOOING ANTI-CHUTE** | |
|---|---|
| Antagoniste du récepteur | |
| des prostaglandines EP-3 | 1,5 g |
| Polyglycéryl 3-hydroxylaurylether | 26 g M.A. |
| Hydroxy propyl cellulose vendue sous la | |
| dénomination de Klucell G par la sté HERCULES | 2 g |
| Conservateur | qs |
| Agoniste des récepteurs aux | |
| prostaglandines EP-2 et/ou EP-4 | 1 g |
| Ethanol 95° | 50 g |
| Aminexil | 0,1 g |
| Eau qsp | 100 g |

Ce shampooing est utilisé quotidiennement à raison de 15 g par chevelure avec un temps de pose de l'ordre d'une minute et ceci pendant une période de 4 mois. On observe alors un ralentissement notable de la chute quotidienne des cheveux.

### Exemple III:

| **GEL ANTI-CHUTE** | |
|---|---|
| Antagoniste du récepteur | |
| des prostaglandines EP-3 | 0,75g |
| Huile essentielle d'Eucalyptus | 1 g |
| Econazole | 0,25g |
| Lauryl polyglyceryl 6 cetearyl glycoléther | 1,9 g |
| Na Glutamate de suif hydrogéné vendu sous la | |
| dénomination ACYLGLUTAMATEHS110 | |
| par la Sté AJINOMOTO | 0,1 g |
| Conservateurs | qs |
| CARBOPOL 934P vendu par la Sté | |
| BF GOODRICH CORPORATION | 0,3 g M.A. |
| Agent de neutralisation | qs pH 7 |
| Eau qsp | 100 g |

Ce gel est appliqué 2 fois par jour (matin et soir) en raison de 25 g sur la totalité du cuir chevelu avec un massage terminal. Après 3 mois d'application la chute quotidienne des cheveux et des poils est clairement ralentie.

### Exemple IV :

| **LOTION ANTICHUTE** | |
|---|---|
| Antagoniste du récepteur | |
| des prostaglandines EP-3 | 0,4 g |
| Propylène glycol | 20 g |
| Ethanol 95° | 50 g |
| Aminexil | 0,1 g |
| Eau qsp | 100 g |

Cette lotion est utilisée de la même manière que l'exemple 1. Les résultats observés sont du même ordre.

### EXPÉRIMENTATION:

Afin d'étudier le comportement du follicule pileux en présence d'antagoniste du récepteur des prostaglandines EP-3, la demanderesse s'est servie de la méthode dite du "cheveu en survie" du brevet FR 9508465 de L'ORÉAL.

À partir d'une biopsie de scalp, une bande de cuir chevelu assez fine a été isolée à l'aide d'un scalpel. Avec une micropince, le tissu adipeux autour des follicules a été éliminé, en évitant d'endommager le bulbe pilaire. Sous un microscope, le follicule a été coupé avec un scalpel pour le séparer de son environnement épidermique et dermique.

Un des fragments obtenus a été maintenu en culture dans du milieu Williams E, à 37°C, en atmosphère humide en présence de 5% de CO₂ et servira de témoin.

Les autres fragments sont mis dans le même milieu de culture en présence d'antagoniste du récepteur des prostaglandines EP-3.

Les fragments en présence des agonistes ainsi maintenus en histoculture s'allongent d'une manière significativement plus importante en comparaison avec le fragment témoin sans antagoniste.

## Revendications

1. Composition cosmétique contenant au moins un antagoniste du récepteur des prostaglandines EP-3 dans un milieu cosmétiquement acceptable.

2. Composition selon la revendication 1, **caractérisée par le fait que** ladite composition cosmétique contient de 0,001 à 10% et de préférence de 0,1 à 5% d'antagonistes en poids par rapport au poids de la composition.

3. Composition selon la revendication 1 ou 2, **caractérisée par le fait que** ladite composition cosmétique contient de 0,001 à 10% et de préférence de 0,1 à 5% d'agonistes des récepteurs des prostaglandines EP-2 et/ou EP-4 en poids par rapport au poids de la composition.

4. Composition selon l'une quelconque des revendications 1 à 3, **caractérisée par le fait que** la composition contient en outre un milieu cosmétiquement acceptable constitué d'eau ou d'eau et d'au moins un solvant organique choisi dans le groupe constitué par les solvants organiques hydrophiles, les solvants organiques lipophiles, les solvants amphiphiles ou leurs mélanges.

5. Composition selon la revendication 4, **caractérisée par le fait que** les solvants organiques sont choisis dans le groupe constitué par les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

6. Composition selon la revendication 4 ou 5, **caractérisée par le fait que** le ou les solvants organiques représentent de 5 à 98% du poids total de la composition.

7. Composition selon l'une quelconque des revendications 1 à 6, **caractérisée par le fait que** la composition comprend en outre au moins une phase grasse.

8. Composition selon la revendication 7, **caractérisée par le fait que** la phase grasse représente de 0 à 50% du poids total de la composition.

9. Composition selon l'une quelconque des revendications 1 à 8, **caractérisée par le fait qu'**elle contient en outre au moins un additif choisi dans le groupe constitué par les gélifiants et/ou épaississants classiques hydrophiles ou lipophiles; des actifs hydrophiles ou lipophiles; des conservateurs; des antioxydants; des parfums; des émulsionnants; des agents hydratants; des agents pigmentants; des dépigmentants; des agents kératolytiques; des vitamines; des émollients; des séquestrants; des tensio-actifs; des polymères; des agents alcanisants ou acidifiants; des charges; des agents anti-radicaux libres; des céramides; des filtres solaires; des répulsifs pour insectes; des agents amincissants; des matières colorantes; des bactéricides; des anti-pelliculaires.

10. Composition selon l'une quelconque des revendications 1 à 9, **caractérisée par le fait que** la composition se présente sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules.

11. Composition selon l'une quelconque des revendications 1 à 10, **caractérisée par le fait que** la composition a l'aspect d'une crème blanche ou colorée, d'une pommade, d'un lait, d'une lotion, d'un sérum, d'une pâte, d'une mousse ou d'un solide.

12. Composition selon l'une quelconque des revendications 1 à 11, **caractérisée par le fait que** la composition présente un pH compris entre 3 et 8.

13. Utilisation d'antagonistes du récepteur des prostaglandines EP-3 comme agent cosmétique permettant d'atténuer, de diminuer ou de stopper la chute des poils et des matières kératiniques.

14. Utilisation d'une composition décrite dans l'une quelconque des revendications 1 à 12 pour atténuer, de diminuer ou d'arrêter la chute des cheveux et des poils.

15. Procédé de traitement cosmétique destiné à atténuer, diminuer ou stopper la chute des cheveux et des poils, **caractérisé en ce qu'**il consiste à appliquer sur les cheveux et les poils une quantité cosmétiquement efficace d'antagonistes du récepteur des prostaglandines EP-3.

16. Procédé de traitement cosmétique destiné à atténuer, diminuer ou stopper la chute des cheveux et des poils, **caractérisé par le fait qu'**il consiste à appliquer sur les cheveux ou les poils une composition cosmétique telle que définie dans l'une quelconque des revendications 1 à 12.
